# EUROPEAN PATENT SPECIFICATION

(11) **EP 3 368 453 B1**
(45) Date of publication and mention of the grant of the patent: **25.05.2022**
(21) Application number: 16860381.9
(22) Date of filing: 14.10.2016
(51) Int. Cl.: B65G 51/26, A61L 2/10

(54) **A DECONTAMINATION/DISINFECTION DEVICE FOR CARRIERS FOR TRANSPORTING RECEPTACLES ETC, AND A METHOD OF DECONTAMINATING AND/OR DISINFECTING CARRIERS**
DEKONTAMINATIONS-/DESINFEKTIONSVORRICHTUNG FÜR TRÄGER ZUM TRANSPORT VON BEHÄLTERN USW., UND EIN VERFAHREN ZUR DEKONTAMINATION UND/ODER DESINFEKTION VON TRÄGERN
DISPOSITIF DE DÉCONTAMINATION/DÉSINFECTION POUR DES SUPPORTS DE TRANSPORT DE RÉCIPIENTS, ENTRE AUTRES, ET PROCÉDÉ DE DÉCONTAMINATION ET/OU DE DÉSINFECTION DE SUPPORTS

(30) Priority: 28.10.2015 SE 1551388
(43) Date of publication of application: 05.09.2018
(73) Proprietor: Graniten Group AB, 451 76 Uddevalla (SE)
(72) Inventor: ERIKSSON, Karl Olof, 451 32 Uddevalla (SE)
(74) Representative: AWA Sweden AB
(86) International application number: PCT/SE2016/050995
(87) International publication number: WO 2017/074242

(56) References cited:
- EP-A1- 1 435 245
- WO-A1-2013/068379
- DE-A1- 4 407 183
- US-A- 4 767 932
- US-A- 6 039 928
- US-A1- 2006 011 263
- US-A1- 2013 264 495
- US-A1- 2015 246 152

## Description

### TECHNICAL FIELD

This technology generally concerns tube systems that are intended for pneumatic transport of articles in special carriers, also called cartridges, and specifically concerns a device as well as a method for performing decontamination and/or disinfection of such carriers in environments where bacteria etc. may contaminate the carriers.

### BACKGROUND

Pneumatic tube transport systems using carriers have for many years been commonly used within many technical areas, such as for transporting documents or articles of different kinds in the carriers and between office areas, as well as in banks. In later years the transport of documents in such pneumatic tube transport carriers has to the greatest extent been replaced by electronic digital document transmission.

However, pneumatic tube transport systems are used more and more for sending articles and objects, such as test tubes and samples of different kinds, between wards or infirmaries in hospitals etc. For such healthcare applications it is a serious task to perform adequate infection control with regard to the pneumatic transport systems. This is essential to provide a healthy environment with adequate safety for patients as well as for hospital personnel. A major problem that may be encountered in association with such applications of pneumatic tube transport systems is that the actual carriers get contaminated by hazardous substances or materials or by medicaments carried therein. Normally, special leak-proof containers are used to transport hazardous material inside the carriers. Even so, it may happen that the inside as well as the outside of carriers by mistake may be contaminated by bacteria, virus or hazardous substances.

In order to deal with such problems it has been known to decontaminate/disinfect the tube systems as well as the carriers by cleaning them using some appropriate liquid cleaning agent, such as chlorine and/or alcohol. For the decontamination of the actual tube systems it has also been known to use special cleaning carriers/cartridges that are sent through the tube systems. Carriages and containers have also been disinfected by first being subjected to some appropriate sterilizing gas and then being soaked in an appropriate germicide solution.

Presently used methods of decontaminating and disinfecting carriers for pneumatic tube transport systems are quite cumbersome and time-consuming and there is thus a clear demand for improvements within this field.

It is known to use ultraviolet radiation in order to sterilise internal and external surfaces of containers and carriers, see e.g. EP1435245 A1, US2015/246152 A1, DE4407183 A1, US4767932 A, US2013/264495 A1, WO2013/068379 A1, US2006/011263 A1, US6039928 A.

### SUMMARY

It is a general object to provide an improved solution to the above discussed problems.

In particular it is an object to suggest improved methods of decontaminating/disinfecting carriers for pneumatic tube transport systems.

In particular it is another object to suggest improved devices for decontaminating/disinfecting carriers for pneumatic tube transport systems.

These and other objects are met by the technology as defined by the accompanying claims.

The technology generally relates to a pneumatic tube system for transporting objects and articles of various kinds between stations of the system.

In a first aspect of the technology, there is provided an improved method of decontaminating and/or disinfecting carriers used for transporting receptacles etc. containing hazardous substances and/or materials between different locations in pneumatic tube transport systems, whereby carriers coming from a sending location and arriving at a destination location are opened at least at one of their ends to remove a transported receptacle therefrom. In a basic configuration the method includes introducing at least one central UV-lamp into the interior of the carriers at the destination location, simultaneously advancing a plurality of external UV-lamps arranged around the outside of the carriers and switching on the central as well as external UV-lamps and maintaining them lit for a specified time before removing the central as well as external UV-lamps from the carriers.

In another aspect of the technology there is provided a decontamination/disinfection device configured to perform the method according to the first aspect. In a basic configuration there is provided a supporting frame with at least one central UV-lamp being supported by the frame and extending therefrom and with a plurality of external UV-lamps supported by the frame and extending therefrom so that the external UV-lamps are supported encircling the central UV-lamp at a distance therefrom.

Preferred further developments of the basic idea as well as embodiments thereof are specified in the dependent subclaims.

Advantages offered by this technology, in addition to those described above, will be readily appreciated upon reading the below detailed description of embodiments of the technology.

### BRIEF DESCRIPTION OF THE DRAWINGS

The technology and its further objects and advantages will be best understood by reference to the following description taken together with the accompanying drawings, in which:
- Fig. 1: is a schematical illustration of a basic embodiment of a pneumatic tube transport system where the present technology may be applied;
- Figs. 2A-B: are likewise schematical illustrations of an embodiment of a carrier of a kind that may be used in the system of Fig. 1;
- Fig. 3: is a schematical perspective view of an embodiment of a decontamination and disinfection device of this technology, as applied to a carrier of Figs. 2A-B; and
- Fig. 4: is a schematical top plan view of the decontamination and disinfection device of Fig. 3 supplemented with a screen.

### DETAILED DESCRIPTION

The technology will now be explained with reference to an exemplifying embodiment of a decontaminating and/or disinfecting device as well as method that are very schematically illustrated in the accompanying drawing figures. The embodiment serves to exemplify the use of the principles of the new technology in an application for decontaminating and/or disinfecting carriers/cartridges used in pneumatic tube transporting systems in hospitals and laboratories etc. It shall be emphasized though that the illustrations serve the purpose of describing a preferred embodiment of the technology and are not intended to limit the technology to details or to any specific field of application thereof. The presently proposed solutions may therefore with only minor adaption be applied to most types of pneumatic tube transport systems.

To overcome disadvantages and problems that are normally encountered within this technical field and that were described in the introduction the present technology now suggests a novel approach for optimizing carrier decontamination/disinfection by enabling a quick, problem-free and inexpensive decontamination and disinfection. In essence this is achieved by facilitating the treatment of the carriages while simultaneously improving the economy and speeding up the handling of the carriages by suggesting a unique combination of using UV (ultraviolet) light to simultaneously decontaminate/disinfect/sterilize the inside as well as the outside of the carriers. The unique design of the suggested configuration provides several essential advantages that include quick, secure and environmentally friendly decontamination, easy handling and low energy consumption. Compared to existing techniques this approach enables very quick, easy and economical carrier decontamination/disinfection increasing the capacity without risking decreased decontamination/disinfection quality.

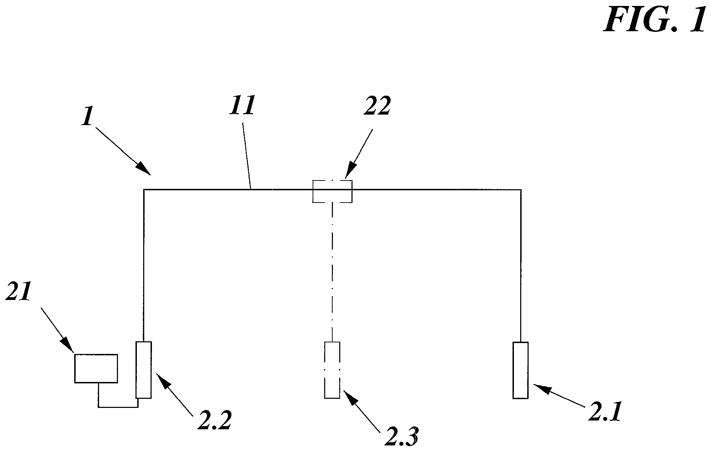

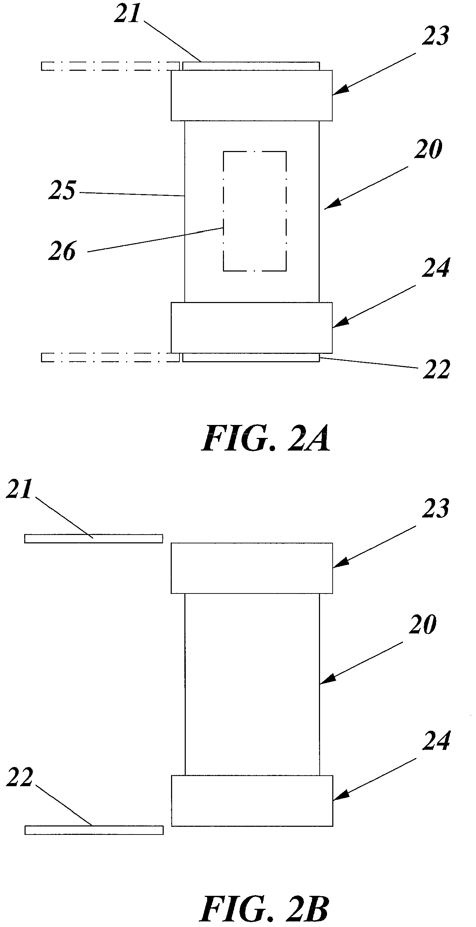

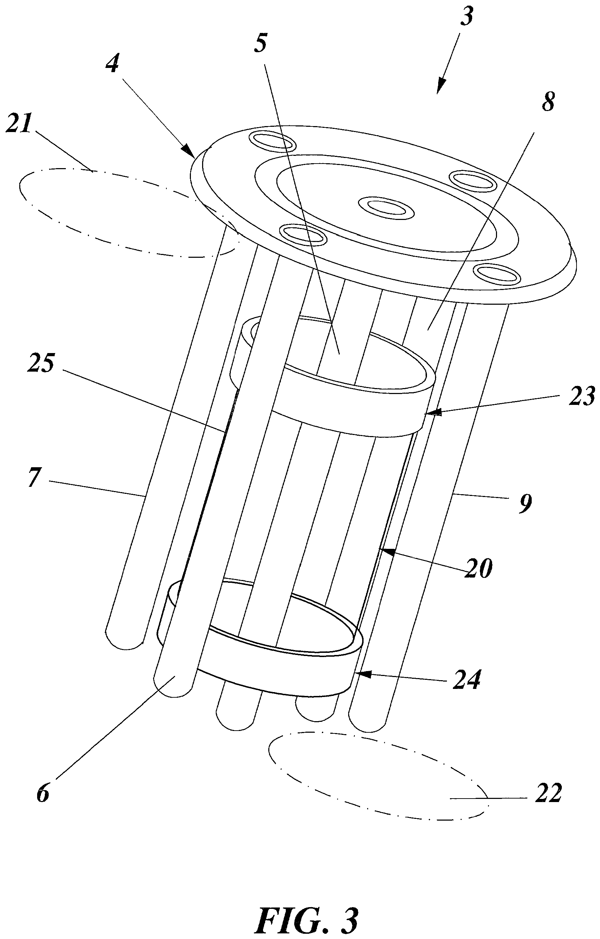

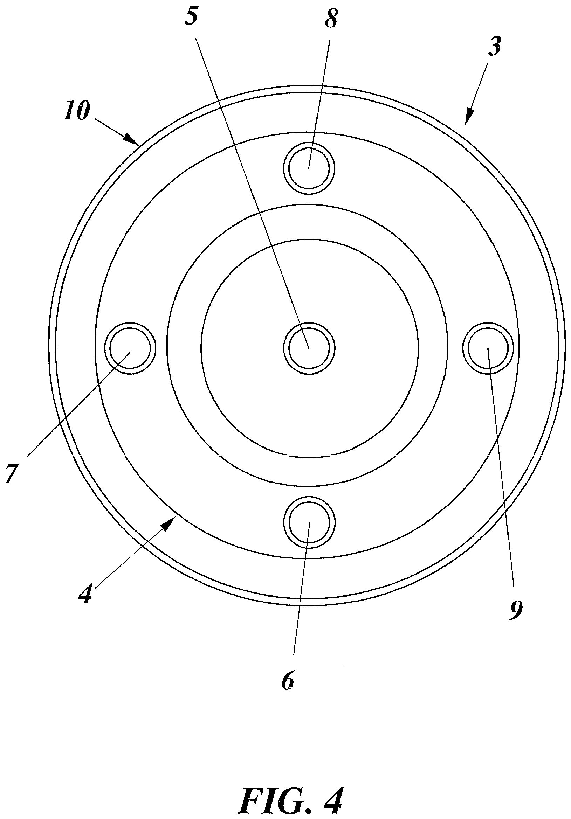

The present technology will now be explained with reference to exemplifying embodiments of the technology that are illustrated in the accompanying drawing figures 1-4. Fig. 1 very schematically illustrates an exemplary embodiment of a basic prior art pneumatic tube transport system 1 of the kind to which this new technology may be applied. The pneumatic tube transport system 1 includes a transport tube 11 that in this basic system transports carriers 20 (see Figs. 2A-B) from a sending station 2.1 to a receiving station 2.2 by vacuum pressure created by a blower 21 or similar apparatus. Such basic pneumatic tube transport systems 1 may for most types of applications be extended and supplemented by adding further sending and/or receiving stations. This is indicated in Fig. 1 by the additional sending station 2.3 that in a conventional manner is connected to the basic transport tube 11 through a switch 22. It is likewise common practice to use some or most stations 2.1-2.3 both as sending and receiving stations by extending the vacuum pressure feeding to the applicable stations.

Figs. 2A-B schematically illustrate an example of basic carriers 20, or cartridges, that are conventionally used to transport articles or documents in pneumatic tube transport systems 1. The carriers 20 conventionally consist of a normally transparent generally tubular cylindrical outer shell 25 being open in at least one end and having a closeable end cap at said at least one end. In the illustrated embodiment the carrier 20 has two open ends, namely an upper end 23 and a lower end 24, with removable end caps 21, 22 provided at each end. As is partly indicated in Figs. 2A and 2B the end caps 21, 22 are unscrewed from the open ends 23, 24 of the outer shell 25A and are then preferably moved away sideways a short distance from said respective open ends 23, 24. A carrier insert (not specifically indicated) may be provided in the carrier 20, inside the cylindrical shell 25 to form a receiving space for transported articles etc. The present technology using any standard type of carrier 20 will allow for the use of different types of conventional additional equipment, such as carrier end cap openers and carrier loaders etc. that are known to skilled practitioners within this general field and that are therefore not illustrated here. The technology may also be applied to many types, shapes and sizes of articles by making any obvious and appropriate adjustments.

However, this technology is specifically intended for pneumatic tube transport systems 1 used for transporting carriers 20 carrying receptacles 26 etc. (schematically indicated in Fig. 2A) containing hazardous substances and/or materials between different sending and/or destination locations 2.1, 2.2, 2.3 of a pneumatic tube transport system 1 in e.g. a hospital environment. For this purpose the technology suggests providing a decontamination or disinfection device 3 having a supporting frame 4. This supporting frame 4 may in the illustrated case, where it cooperates with generally cylindrical carriers 20, have the general shape of a cylindrical disc. The device 3 includes at least one central UV-lamp 5 that is supported by the frame 4 and that in the embodiment extends downwards therefrom. The device 3 further includes a number of, in this embodiment four, external UV-lamps 6-9 that are also supported by the frame 4 and in this embodiment likewise extend downwards therefrom. The external UV-lamps 6-9 are supported by the frame 4 encircling the central UV-lamp 5, at a distance therefrom. As indicated briefly above the number of the central and external UV-lamps may be varied in dependence on the specific application. Similarly, their arrangement or specific mutual positioning may likewise be varied in dependence upon the specific situation and conditions, such as the shape of the carriers of a specific system. As an example, the external UV-lamps may be evenly or unevenly distributed around the central UV-lamp or lamps.

The UV-lamps 5-9 may all have a similar elongated shape and a length that preferably clearly exceeds the length of the carriers 20. This provides for enabling the central 5 and/or external 6-9 UV-lamps to be arranged to light up also an inside of the upper and lower covers 21, 22 at upper and lower carrier 20 ends 23, 24. In order to prevent UV-light from disturbing any activity in spaces surrounding the decontamination/disinfection device 3 a screen 10 may further be provided, surrounding the supporting frame 4 and the central 5 as well as external 6-9 UV-lamps at least during a time when the UV-lamps are lit. According to presently performed calculations and tests the UV-lamps may, depending upon the circumstances have a nominal lamp power of approximately between 25-100Watts to perform adequate decontamination and/or disinfection in the intended hospital environment. Said circumstances would obviously include the degree of carrier contamination, the number of central as well as external UV-lamps in an arrangement and the UV-light exposure time. However, such indications of nominal UV-lamp power only serve to exemplify an application of the technology and shall in no way be regarded as limiting the scope of the technology.

The proposed new technology has been described above with specific reference to presently proposed practical embodiments. However, it should be noted that the technology is in no way restricted to an embodiment where the UV-lamps 5-9 are extended downwards from the supporting frame 4 and are lowered into and around the carriers. It is equally well suited for alternative embodiments where the UV-lamps 5-9 are extending upwards from the supporting frame 4 and are raised into and around the carriers 20. In the same way it is also possible to move either the frame 4 or the carrier 20 in order to extend the UV-lamps 5-9 into and around the carrier 20. The external UV-lamps 6-9 are preferably positioned so as to be evenly distributed around the central UV-lamp 5. Likewise, the central 5 as well as external 6-9 UV-lamps are preferably jointly supported on a common frame 4.

A method of decontaminating/disinfecting carriers 20 used for transporting receptacles etc. containing hazardous substances and/or materials will now be described. The proposed decontamination/disinfection method may depending upon the circumstances be performed by itself or in combination with a conventional periodical cleaning of the carriers. The carriers 20 are transported between the different sending and/or destination locations 2.1, 2.2, 2.3 in the pneumatic tube transport systems 1. Carriers 20 coming from a sending location 2.1 and arriving at a destination location 2.2 are first opened by removing or repositioning covers 21, 22 at upper and/or lower ends 23, 24 thereof to remove a transported receptacle 26 therefrom. According to the presently proposed method a central UV-lamp 5 is in a next method step introduced into the interior of the carriers 20 at the destination location 2.2, e.g. by being lowered into the opened carrier 20.

Simultaneously with introducing the central UV-lamp 5 into the carrier 20 a number of, e.g. four, external UV-lamps 6-9 arranged around the outside of the carrier 20 are advanced, e.g. by being lowered around the opened carrier 20. In a further method step the central 5 as well as external 6-9 UV-lamps are switched on and maintained lit for a specified time period. Said time period during which the central 5 and/or external 6-9 UV-lamps are lit is calculated for each specific application, depending upon the degree of contamination of the carriers 20, the number of and the power of the UV-lamps. This time period will typically be approximately up to one minute and will in normal cases with appropriate UV-lamps and an average degree of contamination of the carriers be between 15-45 seconds and specifically between 15 and 30 seconds. After that time the central 5 as well as external 6-9 UV-lamps are removed, e.g. raised, from the carrier. The procedure may be repeated twice or even several times depending upon the circumstances of a specific application. It should be emphasized though, that the indicated time periods only serve as examples and shall not restrict the technology to any specific length of time of the UV radiation.

The light from the central 5 and/or external 6-9 UV-lamps is preferably also made to illuminate also the inside and possibly the outside of the at least one cover 21, 22 at the upper and/or lower ends 23, 24 of the carriers 20 in order to optimize the decontamination and disinfection. For safety reasons the light from the central 5 and external 6-9 UV-lamps is however preferably screened off from the environment.

In practical tests performed with quite heavily contaminated carriers a number of central and external UV-lamps were used and were arranged generally as disclosed in the specification and drawings. Without radiation the carriers were contaminated with an indefinitely large amount of colony-forming units CFU's. For the radiation UV-lamps were used having a nominal lamp power of approximately 26 Watts and being arranged as specified. In the performed tests this UV-light source provided a radiation of approximately 0,27 W per cm of UV-lamp length and the radiation was performed for a time period of between 15 and 30 seconds. The tests performed with such specifications resulted in a very radical decrease in the number of CFU's being present in the carriers. Specifically, as low numbers as 10-15 CFU's were measured. For even more heavily contaminated carriers a radiation of approximately 0,5 W per cm of UV-lamp length may be considered and for less contaminated carriers a radiation as low as approximately 0,2 W per cm of UV-lamp length may be considered. Once again, these intervals are only given as practical examples and are not intended to restrict the technology in any way.

In alternative, but not specifically illustrated embodiments of the invention variations of the different illustrated parts of the device may be employed without departing from the scope of the invention. Examples of this is the use of optional numbers of central as well as external UV-lamps and/or the use of modified frames for supporting UV-lamps distributed in alternative, possibly irregular, ways. It shall also be emphasized that although the technology has been described and illustrated with reference to an application for transporting hazardous material or substances in hospitals the invention is in no way restricted to such a specific application. The basic principles of the invention may be applied to other types of locations such as laboratories where similar hazardous and contaminating substances and materials are transported.

The present technology has been described in connection with an embodiment that is to be regarded as an illustrative example thereof. It will be understood by those skilled in the art that the present technology is not limited to the disclosed embodiment but is intended to cover various modifications and equivalent arrangements. The present technology likewise covers any feasible combination of features described and illustrated herein. The scope of the present technology is defined by the appended claims.

## Claims

1. A method of decontaminating and/or disinfecting carriers (20) used for transporting receptacles containing hazardous substances and/or materials between different sending and/or destination locations (2.1, 2.2, 2.3) in pneumatic tube transport systems (1), whereby carriers coming from a sending location (2.1) and arriving at a destination location (2.2) are opened by removing or repositioning covers (21, 22) at least at one of their ends (23, 24) to remove a transported receptacle therefrom, **characterized by**:
- introducing at least one central UV-lamp (5) into the interior of the carriers at the destination location;
- simultaneously advancing a plurality of external UV-lamps (6-9) arranged around the outside of the carriers;
- switching on the central as well as external UV-lamps and maintaining them lit for a specified time before removing the central as well as external UV-lamps from the carriers.

2. A method according to claim 1, **characterized in that** the specified time during which the central (5) and/or external (6-9) UV-lamps are lit is calculated for each specific application, depending upon the degree of contamination of the carriers (20), the number of and the power of the UV-lamps.

3. A method according to claim 1 or 2, **characterized by** providing central (5) and/or external (6-9) UV-lamps having a length that clearly exceeds the length of the carriers (20) and arranging them so as to illuminate also at least the inside of the covers (21, 22) at the at least one of the ends (23, 24) of the carriers (20).

4. A method according to any of claims 1-3, **characterized by** providing a screen (10) surrounding the central (5) as well as external (6-9) UV-lamps at least during a time when the UV-lamps are lit, whereby the light from the central (5) and external (6-9) UV-lamps is screened off from the environment.

5. A method according to any of claims 1-4, **characterized by** positioning the external UV-lamps (6-9) so as to be evenly distributed around the central UV-lamp (5).

6. A method according to any of claims 1-5, **characterized by** jointly supporting the central (5) as well as external (6-9) UV-lamps on a common frame (4), thereby enabling the simultaneous introduction of the central UV-lamp into the carrier (20) as well as the advancing of the external UV-lamps being lowered around the opened carrier.

7. A decontamination/disinfection device (3) configured to perform a method according to claim 1, **characterized by:**
- a supporting frame (4);
- at least one central UV-lamp (5) supported by the frame and extending therefrom; a plurality of external UV-lamps supported by the frame and extending therefrom; whereby
- the external UV-lamps being supported encircling the central UV-lamp at a distance therefrom.

8. A decontamination/disinfection device (3) according to claim 7, **characterized by** a screen (10) surrounding the supporting frame (4) and the central (5) as well as external (6-9) UV-lamps at least during a time when the UV-lamps are lit.

9. A decontamination/disinfection device (3) according to claims 7 or 8, **characterized in that** the external UV-lamps (6-9) are evenly distributed around the central UV-lamp (5)

10. A decontamination/disinfection device (3) according to any of claims 7-9, **characterized in that** the UV-lamps have a nominal lamp power of approximately between 25-100Watts.

11. A decontamination/disinfection device (3) according to any of claims 7-10, **characterized in that** the central and/or external UV-lamps have a length that clearly exceeds the length of the carriers (20) and are arranged to light up also at least the inside of upper and lower covers (21, 22) at upper and lower carrier (20) ends (23, 24).

## Patentansprüche

1. Ein Verfahren zum Dekontaminieren und/oder Desinfizieren von Trägern (20), die für den Transport von Behältern, die gefährliche Substanzen und/oder Materialien enthalten, zwischen verschiedenen Sende- und/oder Bestimmungsorten (2.1, 2.2, 2.3) in Rohrpostsystemen (1) verwendet werden, wobei Träger, die von einem Sendeort (2.1) kommen und an einem Bestimmungsort (2.2) ankommen, durch Entfernen oder Neupositionieren von Abdeckungen (21, 22) zumindest an einem ihrer Enden (23, 24) geöffnet werden, um ein transportiertes Gefäß davon zu entfernen, **gekennzeichnet durch:**
- Einführen mindestens einer zentralen UV-Lampe (5) in das Innere der Träger am Bestimmungsort;
- Gleichzeitiger Vorschub einer Vielzahl von externen UV-Lampen (6-9), die um die Außenseite der Träger herum angeordnet sind;
- Einschalten der zentralen und externen UV-Lampen und deren Beibehaltung für eine bestimmte Zeit, bevor die zentralen und externen UV-Lampen aus den Behältern entfernt werden.

2. Verfahren nach Anspruch 1, **dadurch gekennzeichnet, dass** die vorgegebene Zeit, während der die zentralen (5) und/oder externen (6-9) UV-Lampen leuchten, für jede spezifische Anwendung in Abhängigkeit vom Verschmutzungsgrad der Träger (20), der Anzahl und der Leistung der UV-Lampen berechnet wird.

3. Verfahren nach Anspruch 1 oder 2, **dadurch gekennzeichnet, dass** zentrale (5) und/oder externe (6-9) UV-Lampen vorgesehen werden, deren Länge die Länge der Träger (20) deutlich übersteigt, und dass sie so angeordnet werden, dass sie auch mindestens die Innenseite der Abdeckungen (21, 22) an mindestens einem der Enden (23, 24) der Träger (20) beleuchten.

4. Verfahren nach einem der Ansprüche 1 bis 3, **dadurch gekennzeichnet, dass** eine Abschirmung (10) vorgesehen ist, die sowohl die zentrale (5) als auch die externen (6 bis 9) UV-Lampen zumindest während der Zeit, in der die UV-Lampen leuchten, umgibt, wodurch das Licht der zentralen (5) und der externen (6 bis 9) UV-Lampen von der Umgebung abgeschirmt wird.

5. Verfahren nach einem der Ansprüche 1 bis 4, **dadurch gekennzeichnet, dass** die äußeren UV-Lampen (6 bis 9) so angeordnet werden, dass sie gleichmäßig um die zentrale UV-Lampe (5) verteilt sind.

6. Verfahren nach einem der Ansprüche 1 bis 5, **dadurch gekennzeichnet, dass** die zentrale (5) und die externen (6 bis 9) UV-Lampen gemeinsam auf einem Gestell (4) gelagert sind, wodurch das gleichzeitige Einführen der zentralen UV-Lampe in den Träger (20) und das Vorschieben der externen UV-Lampen, die um den geöffneten Träger herum abgesenkt werden, ermöglicht wird.

7. Eine Dekontaminations-/Desinfektionsvorrichtung (3), die zur Durchführung eines Verfahrens nach Anspruch 1 konfiguriert ist, **gekennzeichnet durch:**
- einen Stützrahmen (4);
- mindestens eine zentrale UV-Lampe (5), die von dem Rahmen getragen wird und sich von diesem aus erstreckt;
- eine Vielzahl von externen UV-Lampen, die von dem Rahmen getragen werden und sich von diesem aus erstrecken; wobei
- die äußeren UV-Lampen so angebracht sind, dass sie die zentrale UV-Lampe in einem gewissen Abstand umschließen.

8. Dekontaminations-/Desinfektionsvorrichtung (3) nach Anspruch 7, **gekennzeichnet durch** einen Schirm (10), der den Tragrahmen (4) und die zentralen (5) sowie externen (6-9) UV-Lampen zumindest während der Zeit, in der die UV-Lampen leuchten, umgibt.

9. Dekontaminations-/Desinfektionsvorrichtung (3) nach Anspruch 7 oder 8, **dadurch gekennzeichnet, dass** die äußeren UV-Lampen (6-9) gleichmäßig um die zentrale UV-Lampe (5) verteilt sind.

10. Dekontaminations-/Desinfektionsvorrichtung (3) nach einem der Ansprüche 7 bis 9, **dadurch gekennzeichnet, dass** die UV-Lampen eine Nennlampenleistung von etwa 25 bis 100 Watt haben.

11. Dekontaminations-/Desinfektionsvorrichtung (3) nach einem der Ansprüche 7 bis 10, **dadurch gekennzeichnet, dass** die zentralen und/oder externen UV-Lampen eine Länge haben, die die Länge der Träger (20) deutlich übersteigt, und so angeordnet sind, dass sie auch mindestens die Innenseite der oberen und unteren Abdeckungen (21, 22) an den oberen und unteren Enden (23, 24) der Träger (20) beleuchten.

## Revendications

1. Procédé de décontamination et/ou de désinfection de navettes (20) utilisées pour transporter des récipients contenant des substances et/ou des matériaux dangereux entre différents postes d'envoi et/ou de destination (2.1, 2.2, 2.3) dans des systèmes de transport par tube pneumatique (1), dans lequel des navettes provenant d'un poste d'envoi (2.1) et arrivant à un poste de destination (2.2) sont ouvertes en enlevant ou en repositionnant des couvercles (21, 22) au moins à l'une de leurs extrémités (23, 24) pour en retirer un récipient transporté, **caractérisé par** :
- l'introduction d'au moins une lampe à rayons ultraviolets centrale (5) à l'intérieur des navettes au poste de destination ;
- l'avancement simultané d'une pluralité de lampes à rayons ultraviolets externes (6-9) agencées autour de l'extérieur des navettes ;
- l'allumage des lampes à rayons ultraviolets centrale et externes et leur maintien allumé durant un temps spécifié avant d'enlever les lampes à rayons ultraviolets centrale et externes des navettes.

2. Procédé selon la revendication 1, **caractérisé en ce que** le temps spécifié durant lequel les lampes à rayons ultraviolets centrale (5) et/ou externes (6-9) sont allumées est calculé pour chaque application spécifique, en fonction du degré de contamination des navettes (20) ainsi que du nombre et de la puissance des lampes à rayons ultraviolets.

3. Procédé la revendication 1 ou 2, **caractérisé par** la fourniture de lampes à rayons ultraviolets centrale (5) et/ou externes (6-9) ayant une longueur qui dépasse clairement la longueur des navettes (20) et leur agencement de manière à illuminer aussi au moins l'intérieur des couvercles (21, 22) à ladite au moins une des extrémités (23, 24) des navettes (20).

4. Procédé selon l'une quelconque des revendications 1 à 3, **caractérisé par** la fourniture d'un écran (10) entourant les lampes à rayons ultraviolets centrale (5) et externes (6-9) durant un temps où les lampes à rayons ultraviolets sont allumées, moyennant quoi la lumière des lampes à rayons ultraviolets centrale (5) et externes (6-9) est masquée de l'environnement.

5. Procédé selon l'une quelconque des revendications 1 à 4, **caractérisé par** le positionnement des lampes à rayons ultraviolets externes (6-9) de manière à ce qu'elles soient distribuées uniformément autour de la lampe à rayons ultraviolets centrale (5).

6. Procédé selon l'une quelconque des revendications 1 à 5, **caractérisé par** le support conjoint des lampes à rayons ultraviolets centrale (5) et externes (6-9) sur un châssis commun (4), permettant ainsi simultanément l'introduction de la lampe à rayons ultraviolets centrale dans la navette (20) et l'avancement des lampes à rayons ultraviolets externes abaissées autour de la navette ouverte.

7. Dispositif de décontamination/désinfection (3) configuré pour mettre en œuvre un procédé selon la revendication 1, **caractérisé par** :
- un châssis de support (4) ;
- au moins une lampe à rayons ultraviolets centrale (5) supportée par le châssis et s'étendant depuis celui-ci ;
- une pluralité de lampes à rayons ultraviolets externes supportées par le châssis et s'étendant depuis celui-ci ; moyennant quoi
- les lampes à rayons ultraviolets externes sont supportées en encerclant la lampe à rayons ultraviolets centrale à une certaine distance de celle-ci.

8. Dispositif de décontamination/désinfection (3) selon la revendication 7, **caractérisé par** un écran (10) qui entoure le châssis de support (4) et les lampes à rayons ultraviolets centrale (5) et externes (6-9) au moins durant un temps où les lampes à rayons ultraviolets sont allumées.

9. Dispositif de décontamination/désinfection (3) selon la revendication 7 ou 8, **caractérisé en ce que** les lampes à rayons ultraviolets externes (6-9) sont distribuées uniformément autour de la lampe à rayons ultraviolets centrale (5).

10. Dispositif de décontamination/désinfection (3) selon l'une quelconque des revendications 7 à 9, **caractérisé en ce que** les lampes à rayons ultraviolets ont une puissance de lampe nominale comprise entre approximativement 25 watts et 100 watts.

11. Dispositif de décontamination/désinfection (3) selon l'une quelconque des revendications 7 à 10, **caractérisé en ce que** les lampes à rayons ultraviolets centrale et/ou externes ont une longueur qui dépasse clairement la longueur des navettes (20) et sont agencées pour éclairer aussi au moins l'intérieur des couvercles supérieur et inférieur (21, 22) aux extrémités supérieure et inférieure (23, 24) de la navette (20).
